# EUROPEAN PATENT SPECIFICATION

(11) **EP 4 320 625 B1**
(45) Date of publication and mention of the grant of the patent: **07.10.2026**
(21) Application number: 21724637.0
(22) Date of filing: 07.05.2021
(51) Int. Cl.: G16H 20/30, A61B 5/024, G16H 40/63

(54) **METHOD AND DEVICE FOR QUANTIFYING STRENUOUSNESS OF A PHYSICAL EXERCISE SESSION OF A PERSON**
VERFAHREN UND VORRICHTUNG ZUR QUANTIFIZIERUNG DER ANSTRENGUNG EINER KÖRPERLICHEN ÜBUNGSSITZUNG EINER PERSON
PROCÉDÉ ET DISPOSITIF DE QUANTIFICATION DE L'INTENSITÉ D'UNE SÉANCE D'EXERCICE PHYSIQUE POUR UN INDIVIDU

(43) Date of publication of application: 14.02.2024
(73) Proprietor: Huawei Technologies Co., Ltd., Shenzhen, Guangdong 518129 (CN)
(72) Inventor: RINTALUOMA, Tero, 16440 Kista (SE); WIKLUND, Petri, Kristian, 16440 Kista (SE)
(74) Representative: Maiwald GmbH
(86) International application number: PCT/EP2021/062108
(87) International publication number: WO 2022/233423

(56) References cited:
- US-A1- 2015 209 615

## Description

### TECHNICAL FIELD

The disclosure relates to a method of quantifying strenuousness of a physical exercise session, and more particularly to a device for calculating an overall training stress resulting from a training session for a person.

### BACKGROUND

According to the existing arts, exercise and physical activity induced physiological stress monitoring based on Excess Post-Exercise Oxygen Consumption (EPOC). EPOC is defined as the excess oxygen consumed during recovery from exercise as compared to resting oxygen consumption. EPOC accumulates rapidly during intense exercise and is believed to reflect the exercise-induced disturbance of body's homeostasis. However, EPOC-based training stress monitoring is biased towards continuous high-intensity training with much smaller training stress given for longer endurance exercises that is of lower intensity. To monitor the low-intensity training, the conventional techniques describe a method to quantify the low-intensity training-induced training stress as a product of training volume (duration of exercise), and training intensity measured as heart rate level. To quantify the overall training stress, the existing art describes a method of combining the methods described above.

These methods however possess various limitations. Firstly, the training stress calculation does not take into account the effect of pre-existing (residual) fatigue from previous training sessions or accumulated (added) fatigue on training stress. Secondly, the existing methods do not take into account the sports type-specific training stress accumulation. For example, the EPOC and relative heart rate-based training stress estimation can be different for running, cycling, and cross-country skiing due to use of different muscle groups and biomechanics in each sports type.

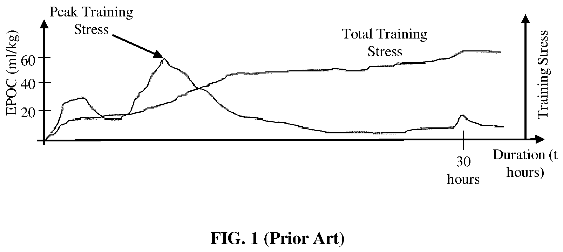

FIG. 1 is an exemplary representation of training stress accumulation, in accordance with a prior art. FIG. 1 illustrates how the peak training stress is estimated from the peak EPOC and total training stress based on training volume (i.e. duration) and intensity based on heart rate level. Total training stress/effect is calculated from the peak training stress/effect (peakTE=peakTE(maxstress),
where maxstress=maxstress (HRReff, maxstress)) and the base training stress/effect (baseTE=baseTE (Σw i*HRReffi)). However, the training stress estimation according to FIG. 1, does not consider the training history and associated accumulated fatigue, current performance level, and sports type, all of which contribute to overall stress caused by exercise. Further, according to the existing arts, training stress is calculated separately for high and low-intensity exercises.

US 2015/209615 A1 describes a zoning method of processing threshold and metabolic and heart rate training data and sensors and apparatus for displaying the same.

Therefore, there arises a need to address the aforementioned technical drawbacks in known techniques or technologies in capturing the true physiological stress of training.

### SUMMARY

It is an object of the disclosure to provide an improved method of quantifying strenuousness of a physical exercise session and a device for calculating an overall training stress resulting from a training session for a person while avoiding one or more disadvantages of prior art approaches.

These objects are achieved by the features of the independent claims. Further, implementation forms are apparent from the dependent claims, the description, and the figures.

The disclosure provides a method of quantifying strenuousness of a physical exercise session and a device for calculating an overall training stress resulting from a training session for a person.

According to a first aspect, there is provided a method of quantifying strenuousness of a physical exercise session. The method includes measuring a person's heart rate during the physical exercise session. The method includes, for each respective heart rate zone among several contiguous heart rate zones, calculating a strenuousness value as a function fᵢ of a product wᵢ*zᵢ of a weight value wᵢ and a duration zᵢ, the duration zᵢ being the total time zᵢ in which the measured heart rate is in the respective heart rate zone. The method includes summing the strenuousness values fᵢ of the heart rate zones.

Thus, the training stress is estimated by first classifying the heart rate sample during a training session into different heart rate zones based on user's maximum heart rate. The overall training stress estimation is improved by calculating the fatigue level based on the measured performance level during the endurance exercise and using the calculated fatigue level scaling coefficient to adjust the training stress and recovery time accordingly. Furthermore, the fatigue level scaling coefficient can be adapted individually for each user and thus provide more personalized training stress and recovery time values.

Optionally, the weight value wᵢ is determined based on the heart rate zone and a duration tₜₒₜ of the physical exercise session. Optionally, each strenuousness value is calculated in dependence of a fitness level calculation function flᵢ for the respective heart rate zone.

According to the first aspect, the overall training stress is calculated based on the sum of all strenuousness values divided by the total duration tₜₒₜ which is the sum of the time spend in all heart-rate zones. According to the first aspect, the overall training stress is adjusted by a fatigue level coefficient which is an estimate of a fatigue level of the person based on a work metric, such as work to effort ratio, recorded during the session. Optionally, the fatigue level coefficient is also calculated based on the residual and/or added fatigue estimated from the real-time performance level during the training session.

According to the first aspect, alternatively or additionally, the overall training stress is adjusted by a sport type calculation function depending on the type of sports practice in the training session, to account for the physiological strain perceived by the person to quantify the relative intensity and duration of the training session. Thus, sport type-specific scaling coefficient is used to better match the perceived training stress between different sport types. For example, in sports types such as running where the user needs to make a lot of effort to carry his/her own weight accumulates fatigue faster than in cycling or cross-country skiing.

Optionally, the overall training stress is calculated according to the equation $f = \frac{{\sum }_{i} {w}_{i} {z}_{i} {fl}_{i}}{{t}_{tot}} \times flc \times stc$. Here, wᵢ is the weight calculation function for heart rate zone i, zᵢ is the duration of the time spent in heart rate zone i , flᵢ is the fitness level calculation function for heart rate zone i, tₜₒₜ is the sum of the time spent in all heart rate zones, flc is the fatigue level calculation function and stc is the sport type calculation function, and where one or more of flᵢ, flc and stc may be set to 1.0. Optionally, the method includes calculating a recovery time for the person based on the overall training stress.

According to a second aspect, there is provided a computer program for use in a mobile or wearable fitness tracker device. The computer program includes computer-readable code which, when run in a processor, will cause the processor to perform the above method. Optionally, the computer program further includes a non-transitory storage device holding the computer-readable code means.

According to a third aspect, there is provided a device for calculating an overall training stress resulting from a training session for a person. The device includes a processor and a program memory. The program memory stores the above computer program so that it can be executed in the processor.

The device enables to improve overall training stress and recovery time estimation by taking into account the measured fatigue level. Personally fitted fatigue level scaling parameters take into account a person's individual physiological characteristics better than general fitness-based models in the prior art. Further, the base training stress calculation is computationally inexpensive.

Optionally, the device includes at least one input for receiving real-time information about the heart rate of the person and optionally at least one of the following types of sensor data for use by the computer program: speed sensor data, power sensor data, accelerometer data and electromyography data.

Optionally, the device includes at least one input for receiving at least one of the following types of data for use by the computer program: fitness level of the person, type of sport performed in the session, residual recovery time of the person, as the remaining recovery time after a previous training session, baseline performance level as a long-term indicator of the person's aerobic performance capacity, maximum heart rate of the person, resting heart rate and heart rate zones.

Optionally, the device comprises a data memory holding at least one of the following types of data for use by the computer program: fitness level of the person, type of sport performed in the session, residual recovery time of the person, as the remaining recovery time after a previous training session, baseline performance level as a long-term indicator of the person's aerobic performance capacity, maximum heart rate of the person, resting heart rate and heart rate zones.

A technical problem in the prior art is resolved, where the technical problem concerns are inaccuracy in calculating the overall training stress.

Therefore, in contradistinction to the prior art, according to the method and the device of the disclosure, overall training stress estimation is improved by calculating the fatigue level based on measured performance level during the endurance exercise and by using the calculated fatigue level scaling coefficient to adjust the training stress and recovery time accordingly. Furthermore, the fatigue level scaling coefficient can be adapted individually for each user and thus provide more personalized training stress and recovery time values. Further, the disclosure can be implemented as a software component into portable devices such as mobile phones, watches, or fitness trackers capable of measuring heart rate and/or speed, power, or other exercise intensity information. The method calculates the overall training stress while also taking into account the measured residual fatigue of previous exercises, the current performance level, and sport type, thereby improving the accuracy in calculating the true physiological stress of training.

These and other aspects of the disclosure will be apparent from and the implementation(s) described below.

### BRIEF DESCRIPTION OF DRAWINGS

Implementations of the disclosure will now be described, by way of example only, with reference to the accompanying drawings, in which:
FIG. 1 is an exemplary representation of training stress accumulation, in accordance with a prior art;
FIG. 2 is a block diagram illustrating an environment for performing overall training stress estimation, in accordance with an implementation of the disclosure;
FIG. 3 is a block diagram of a device for calculating an overall training stress resulting from a training session for a person, in accordance with an implementation of the disclosure;
FIG. 4 is an exemplary illustration of a real-time performance level for an exercise session, in accordance with an implementation of the disclosure;
FIG. 5 is an exemplary illustration of fitness level and fatigue level adjusted recovery time, in accordance with an implementation of the disclosure;
FIG. 6 is a flow diagram that illustrates a method of quantifying a strenuous physical exercise session, in accordance with an implementation of the disclosure; and
FIG. 7 is an illustration of an exemplary computing arrangement that is used in accordance with implementations of the disclosure.

### DETAILED DESCRIPTION OF THE DRAWINGS

Implementations of the disclosure provide a method of quantifying strenuousness of a physical exercise session and a device for calculating an overall training stress resulting from a training session of a person.

To make solutions of the disclosure more comprehensible for a person skilled in the art, the following implementations of the disclosure are described with reference to the accompanying drawings.

Terms such as "a first", "a second", "a third", and "a fourth" (if any) in the summary, claims, and foregoing accompanying drawings of the disclosure are used to distinguish between similar objects and are not necessarily used to describe a specific sequence or order. It should be understood that the terms so used are interchangeable under appropriate circumstances, so that the implementations of the disclosure described herein are, for example, capable of being implemented in sequences other than the sequences illustrated or described herein. Furthermore, the terms "include" and "have" and any variations thereof, are intended to cover a non-exclusive inclusion. For example, a process, a method, a system, a product, or a device that includes a series of steps or units, is not necessarily limited to expressly listed steps or units but may include other steps or units that are not expressly listed or that are inherent to such process, method, product, or device.

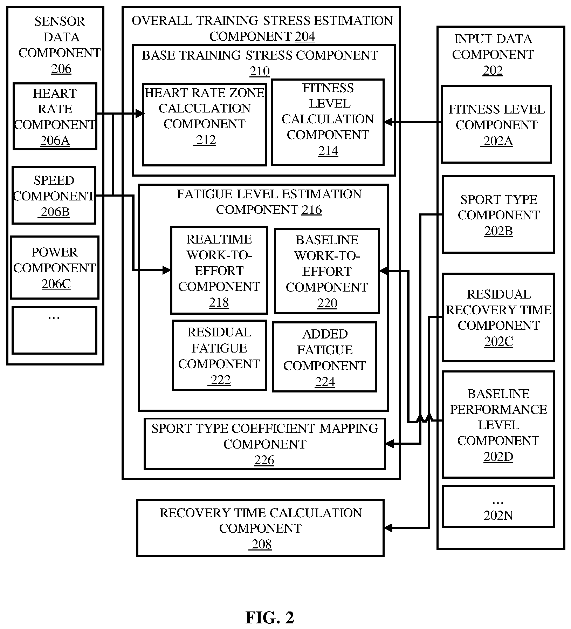

FIG. 2 is a block diagram illustrating an implementation system for performing overall training stress estimation, in accordance with an implementation of the disclosure. The block diagram includes an input data component **202,** an overall training stress estimation component **204,** a sensor data component **206,** and a recovery time calculation component **208.** The input data component **202** includes a fitness level component **202A,** a sport type component **202B,** a residual recovery time component **202C,** and a baseline performance level component **202D.** The sensor data component **206** includes a heart rate component **206A,** a speed component **206B,** and a power component **206C.** The overall training stress estimation component **204** includes a base training stress component **210,** a fatigue level estimation component **216,** and a sport type coefficient mapping component **226.** The base training stress component **210** includes a heart rate zone calculation component **212** and a fitness level calculation component **214.** The fatigue level estimation component **216** includes a real-time work-to-effort component **218,** a baseline work-to-effort component **220,** a residual fatigue component **222,** and an added fatigue component **224.**

The sensor data component **206** and the input data component **202** provide the needed real-time and non-real time inputs to the overall training stress estimation component **204** which process the data and provides the overall training stress estimate as an output. The overall training stress estimate is given to the recovery time calculation component **208** which calculates recovery time estimate based on an overall training stress score.

When a new exercise session is started, the input data is provided to the overall training stress estimation component **204** together with a constant flow of sensor data samples from the sensor data component **206.** On receiving the input data, the fitness level component **202A** may read the fitness level information from a RAM or a non-volatile memory and sends the fitness level information to the fitness level calculation component **214.** Optionally, the sport type component **202B** sends current sport type information to the sport type coefficient mapping component **226.** Optionally, the residual recovery time component **202C** reads residual recovery time information from the RAM or the non-volatile memory and sends the residual recovery time information to the recovery time calculation component **208.** Optionally, the baseline performance level component **202D** reads sport type-specific work-to-effort baseline from the RAM or the non-volatile memory and sends the sport type-specific work-to-effort baseline to the baseline work-to-effort component **220.** Optionally, the heart rate component **206A** starts to send heart rate samples periodically (together with the corresponding timestamps) to the heart rate zone calculation component **212** and the real-time work-to-effort component **218.** Optionally, the speed component **206B** starts to send speed samples periodically (together with the corresponding timestamps) to the real-time work-to-effort component **218.** Optionally, the power component **206C** starts to send power samples periodically (together with the corresponding timestamps) to the real-time work-to-effort component **218.**

The heart rate zone calculation component **212** may classify heart rate samples to heart rate zones and updates a corresponding heart rate zone duration (zi) with a sample interval. Further, the heart rate zone calculation component **212** updates its zone specific dynamic weighing coefficients (wᵢ) as a function of the accumulated total duration (tₜₒₜ). The fitness level calculation component **214** calculates fitness level scaling coefficient (flᵢ) for each heart rate zone (i). When the exercise session ends the base training stress component **210** calculates the base part as "(∑wᵢ zᵢ flᵢ)/ tₜₒₜ" of the overall training stress estimation function.

Full overall training stress calculation formula is shown in Equation 1 as: $Overall training stress = \left(\sum {w}_{i} {z}_{i} {fl}_{i}\right) / {t}_{tot} * fatigueLevelCoefficient * sportTypeCoefficient ;$ Equation (1)

Where wᵢ is the result of the weight calculation function for the heart rate zone (i), zᵢ is the duration in seconds user spent in the heart rate zone (i), flᵢ is the result of the fitness level calculation function for the heart rate zone (i), tₜₒₜ is the total accumulated duration in seconds of all heart rate zones, fatigueLevelCoefficient is the result of the fatigue level estimation function or process, sportTypeCoefficient is the result of sport type coefficient mapping function and Overall training stress is the perceived training stress for a given exercise.

These parameters can be used to estimate the overall training stress, which indicates the strenuousness of the single exercise session. The exercise session is defined as an activity performed by the user e.g. running and it starts when the user begins to run and ends when the user stops running. Training stress estimates the impact of single-session training stress on the user's future aerobic performance capacity. One output for the user is the overall training stress score which can be given as a numerical value, for example, ranging from 1.0 to 5.0

The real-time work-to-effort component **218** calculates the real-time performance level as a work-to-effort ratio of the speed over heart rate (i.e. speed/HR). Optionally, work-to-effort ratio calculation can use the power as an input and the work-to-effort ratio is calculated as power/HR. The baseline work-to-effort component **220** calculates long term stable work-to-effort ratio for each supported sport type. The residual fatigue component **222** estimates the user's residual fatigue level from the current performance level calculated based on information provided by the real-time work-to-effort **218** and baseline work-to-effort **220** components. The added fatigue component **224** further estimates the accumulated fatigue of the current exercise. When the exercise session ends, the fatigue level estimation component **216** calculates the final fatigue level scaling coefficient. The sport type coefficient mapping component **226** prepares the sport type-specific scaling coefficient for the overall training stress estimation.

When the exercise session ends the overall training stress estimation component **204** calculates the overall training stress from the results of the base training stress component **210,** the fatigue level estimation component **216,** and the sport type coefficient mapping component **226.** A corresponding overall training stress calculation formula is shown in Equation 1. The recovery time calculation component **208** calculates the estimated recovery time needed for the user's body to fully recover after the exercise session. Recovery time calculation uses the overall training stress value as an input for the recovery time estimation.

According to the disclosure herein, the overall training stress is improved by taking better into account the perceived training stress of the low-intensity endurance exercises. This improvement is implemented in the base training stress estimation component **204.** Also, the base training stress calculation is computationally less intensive than the prior art solutions.

Training stress is further improved by using the measured real-time fatigue level information of the user. Fatigue level estimation is implemented in the fatigue level estimation component **216.** Optionally, fatigue level estimation parameters can be personalized for each user by the same optimization rules that are used to define generic fatigue level estimation parameters.

Overall training stress calculation is additionally improved with the sport type-specific scaling coefficient. This is implemented in the sport type coefficient mapping component **226.** Since recovery time estimation is done based on the overall training stress score, the accuracy of the recovery time estimation is also improved. Recovery time calculation is implemented in the recovery time calculation component **208.**

Table 1 below shows how numerical values correspond to the training stress. Another output for the user can be the estimated recovery time in minutes which is calculated based on the overall training stress. In addition, the fatigue level information may be provided to the user.

**Table 1. Training stress**

| Overall training stress | Training stress |
|---|---|
| Score | |
| 1 | Very small |
| 2 | Small |
| 3 | Moderate |
| 4 | Large |
| 5 | Very large |

The training stress is estimated by first classifying the heart rate sample during the training session into one of five heart rate zones based on the user's maximum heart rate as shown in Table 2.

**Table 2. Heart rate zones**

| HR zone | %HRmax range |
|---|---|
| Zone 1 | 50-59% |
| Zone 2 | 60-69% |
| Zone 3 | 70-79% |
| Zone 4 | 80-89% |
| Zone 5 | 90-100% |

Duration spent in each heart rate zone (zᵢ) is multiplied by the corresponding dynamic weighing coefficient (wᵢ) and then summed up and divided by the total accumulated zone duration (tₜₒₜ). Weighing coefficient (wᵢ) is dynamic coefficient that is calculated as function of duration. The weight (wᵢ) can be a result of the piece wise linear function or other suitable polynomial function. For example, the weighing coefficient (wᵢ) is calculated as a function of accumulated total zone duration (tₜₒₜ) starting from the beginning of the exercise. The value of (w₃), the coefficient for heart rate zone 3 at time instance (t), can be for example 3.4. Corresponding zone duration for heart rate zone 3 (z₃) can be, for example, 1200 seconds.

If the user's aerobic fitness level information i.e. VO2Max or vVO2Max in addition to personal information such as age and gender are available for training stress calculation, then the fitness level coefficient (flᵢ) can be calculated and the fitness level coefficient (flᵢ) can be used to scale training stress accordingly. The fitness level coefficient is applied because similar training loads may have different effects on homeostasis in well-trained and untrained individuals. Typically, a person with a high fitness level requires a greater absolute training load than a low fit person to induce relatively similar training stress. The training stress is scaled based on fitness level indexed by VO2Max value (or vVO2max value), gender, and age. For example, the fitness level coefficient for heart rate zone 1 for a 25-year-old female with a VO2max value of 40 milliliter per kilogram per minute (ml/kg/min) can be fl₁ = 1.5 and for a 25-year-old female with VO2max value 50 ml/kg/min the coefficient can be fl₁ = 1.35, indicating that a person with a 20% lower VO2max may have approximately 15% higher relative training stress from the same absolute training load. The fitness level coefficient (flᵢ) can be different for each heart rate zone or it can be the same for all zones. If the fitness level coefficient is not available or cannot be calculated, then the default value 1.0 is used.

The base training stress part of the Equation 1 (in component (210)) is then calculated by (∑wᵢ zᵢ flᵢ)/ tₜₒₜ, more specifically (w₁ z₁ fl₁ + w₂ z₂ fl₂ + w₃ z₃ fl₃ + w₄ z₄ fl₄ + w₅ z₅ fl₅)/ tot.

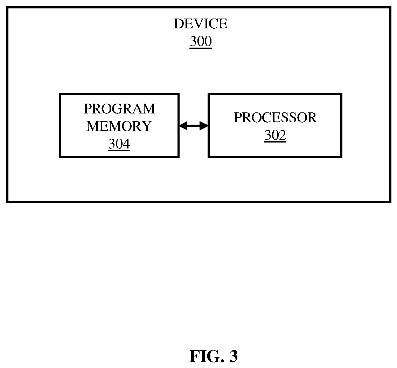

FIG. 3 is a block diagram of a device **300** for calculating an overall training stress resulting from a training session for a person, in accordance with an implementation of the disclosure. The device **300** includes a processor **302** and a program memory **304.** The program memory **304** holding a computer program so that it can be executed in the processor **302.** The program memory **304** is a non-transitory computer-readable storage media encoded a computer-readable code means, which can be executed by the processor **302** to calculate overall training stress.

The device **300** measures a person's heart rate during the physical exercise session. The device **300,** for each respective heart rate zone among several contiguous heart rate zones, calculates a strenuousness value as a function fᵢ of a product wᵢ*zᵢ of a weight value wᵢ and a duration zᵢ, the duration zᵢ being the total time zᵢ in which the measured heart rate is in the respective heart rate zone. The device **300** sums the strenuousness values fᵢ of the heart rate zones. The device **300** enables to improve overall training stress and recovery time estimation by taking into account the measured fatigue level. Personally fitted fatigue level scaling parameters take into account a person's physiological characteristics better than general fitness-based models in the prior art. Further, the base training stress calculation is computationally inexpensive

Optionally, the device **300** includes at least one input for receiving real-time information about the heart rate of the person. Optionally, at least one of the following types of sensor data for use by the computer program: speed sensor data, power sensor data, accelerometer data and electromyography data.

Optionally, the device **300** further includes at least one input for receiving at least one of the following types of data for use by the computer program: a fitness level of the person, type of sport performed in the session, residual recovery time of the person, as the remaining recovery time after a previous training session, baseline performance level as a long-term indicator of the person's aerobic performance capacity, a maximum heart rate of the person, a resting heart rate and heart rate zones.

Optionally, the device **304** further includes a data memory holding at least one of the following types of data for use by the computer program: a fitness level of the person, type of sport performed in the session, residual recovery time of the person, as the remaining recovery time after a previous training session, baseline performance level as a long-term indicator of the person's aerobic performance capacity, maximum heart rate of the person, resting heart rate and heart rate zones.

The device **300** may be a mobile device or a wearable fitness tracker device. The device **300** can be attached to any skin area on the body of the user. The device **300** may be wearable around a shoulder, an ear, the neck, a finger, a palm, an ankle, the chest, an arm, the waist, a leg, a foot, or the torso. The device **300** may include an adaptor that allows the device **300** to be reversibly connected to gym equipment, an exercise apparatus, a sport gear, or the like.

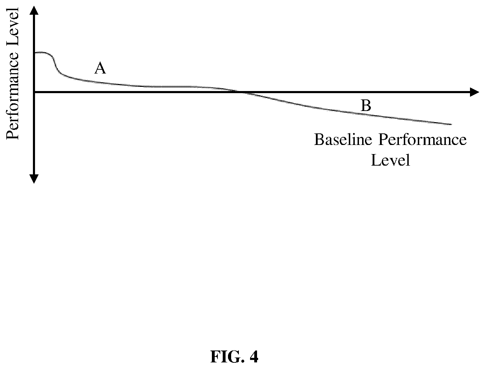

FIG. 4 is an exemplary illustration of a real-time performance level for an exercise session, in accordance with an implementation of the disclosure. The exemplary illustration illustrates the real-time performance level for an exercise session as work-to-effort ratio and its relation to a baseline performance level. A Fatigue Level Coefficient is a scaling coefficient that is a result of a fatigue level estimation function. The Fatigue level estimation may calculate a real time work-to-effort ratio based on input heart rate and speed by taking an input sample pair and dividing a speed sample with corresponding a heart rate sample i.e. speed/heart rate. The exemplary illustration depicts the calculated work to effort ratio, which is called a performance level or the real-time performance level. The baseline performance level may be the user's sport type-specific long term performance level (i.e. work-to-effort ratio). The performance level calculation can use also some other "work" metric such as power to calculate the work-to-effort ratio.

Optionally, residual fatigue is estimated from the real-time performance level after heart rate level has stabilized for required work intensity. The residual fatigue may be calculated when the body has reached a steady-state, defined as a balance achieved between physiological demand of exercise and body's response to those demands, i.e. in the case of running unchanging heart rate level for the current constant running speed. This happens usually after about 10 minutes from the beginning of the exercise but before cardiac drift starts to decrease performance level. The residual fatigue may be caused by unresolved fatigue caused by the previous training sessions and/or other physiological stress caused by for example inadequate sleep. This is marked as section (A) in FIG. 4. If the measured performance level is below the baseline level or other selected threshold level. Then, the body is not yet recovered from the previous exercises. For example, if the measured performance level is -5% from the baseline level, then the body is not fully recovered.

Further, added fatigue is estimated from the real-time performance level over the whole current exercise session. The added fatigue is the level of exhaustion due to strenuous activity or exercise. This is marked as section (B) in FIG. 4. For example, the added fatigue can be estimated by calculating the integral of real-time performance level and the baseline (or other defined threshold level). The added fatigue can be calculated as a ratio of duration above and below the baseline performance level. Optionally, the added fatigue is to compare added fatigue to the measured residual fatigue. Optionally, an average performance level value is used as a measure of the added fatigue. For example, if the measured performance level decreases further from the performance level at the beginning of the exercise then this is contributing into the added fatigue. Optionally, if the performance level is higher at the end of the exercise than at the beginning of the exercise, then this is not further contributing into fatigue level.

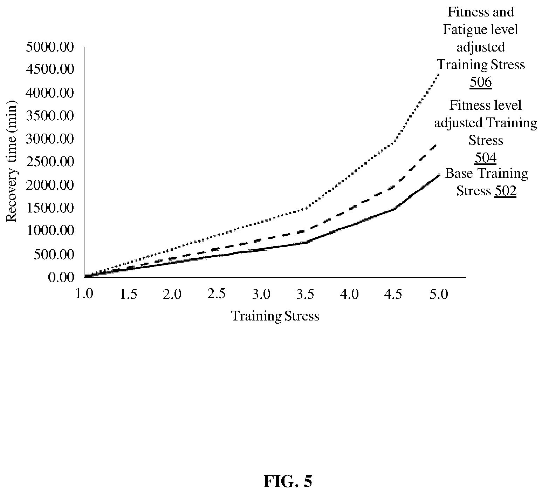

FIG. 5 is an exemplary illustration of a fitness level and a fatigue level adjusted recovery time, in accordance with an implementation of the disclosure. The exemplary illustration depicts training stress plotted on an X-axis and recovery time plotted on a Y-axis. The training stress includes, for instance, base training stress **502,** fitness level adjusted training stress **504,** and fitness and fatigue level adjusted training stress **506.**

Optionally, a fatigueLevelCoefficient is estimated based on calculated parameters such as residual fatigue and added fatigue. For example, if the measured residual fatigue level is at -5% below the baseline for a relatively short exercise (~30 minutes) and fatigue continues to accumulate (i.e. added fatigue), then the fatigueLevelCoefficient may be greater than 1.0, i.e. it may be 1.3. In an example, when a user makes a long endurance run with no residual fatigue but with a lot of added fatigue, then the fatigue Level Coefficient may be greater than 1.0. However, if there is no residual fatigue and the performance level is high during the exercise and the added fatigue accumulates slowly then the fatigue Level Coefficient may be less than 1.0 and would scale down the base training stress **502** value, and thus the needed recovery time is also scaled down.

Optionally, the fatigue estimation function can be implemented with a trained neural network or by a multi-parameter mathematical function derived from statistical analysis. Optionally, inputs for the fatigue estimation are, but not limited to, residual fatigue and added fatigue. Optionally, an output of the fatigue estimation function is the fatigue Level Coefficient.

For example, in the simplest form, the estimation function is calculated as: $Fatigue Level Coefficient = X 1 * residual Fatigue + X 2 * added Fatigue$ where X1 and X2 are constants derived from the statistical analysis. Optionally, the fatigue level parameter is provided as a direct parameter in estimating the training stress **502,** as compared against the existing methods.

One objective measure for the needed recovery time after an exercise is the duration of rest needed for the body to recover back to the performance level (or above) of the user's baseline performance level. Optionally, the recovery time is calculated based on the overall training stress value. The recovery time may be used to identify optimal parameters for the fatigue level estimation function. Optionally, a neural network for fatigue level estimation can be trained with the optimization rules and historical exercise data derived from the user.

The optimization rules and historical exercise data derived from the user may be defined as follows:
- If the residual recovery time from the previous exercise sessions is recorded as zero but the measured residual fatigue level is greater than zero, then the overall training stress score and corresponding recovery time of the previous exercise session are recorded as too low. Residual recovery time is the remaining recovery time from the previous exercise sessions when the current exercise starts. For example, if the recovery time of the previous exercise session is 30 hours and the time between the current and previous exercise session is 24 hours, then the residual recovery time is 6 hours (30 - 24 = 6).
- If the residual recovery time from the previous exercise sessions is greater than zero but the measured residual fatigue level is less than zero, then the overall training stress score and the recovery time of the previous exercise session are recorded as too high.
- If the residual recovery time from the previous exercise sessions is zero just before the next exercise session and the measured residual fatigue level is zero, then the homeostasis was achieved during the recovery time and optimal parameters are found.

Optionally, the sport type coefficient is the scaling coefficient that is used to adjust the training stress, so that the user's perceived overall training stress would match between different sport types. Different sport types are using different muscle groups and for example, oxygen intake and heart rate levels can be higher for sport types that are using more muscles, but perceived overall training stress can be still lower. For example, in cross-country skiing, athletes upper body engages largely as compared to running, but perceived overall training stress for same exercise duration and heart rate levels can be lower than in running. For example, if sport type coefficient is 1.0 for running, then it can be 0.95 for the cross country skiing, 0.93 for cycling, and 1.1 for swimming. The sport type coefficient can be implemented by using, for example, table lookup or dictionary to get the scaler coefficient for the given sport type. If the sport type is unknown or doesn't have the specific scaling coefficient, then the default value 1.0 is used. In the prior art the different sport types are not taken into account directly in the training stress estimation.

Optionally, the parameter optimization for the fatigue level coefficient calculation can be used to personalize the coefficient for individual user. Optionally, the parameter optimization for the fatigue level coefficient calculation can be used in online machine learning. If the fatigue level cannot be accurately estimated for the given exercise session, the default scaling coefficient 1.0 can be used.

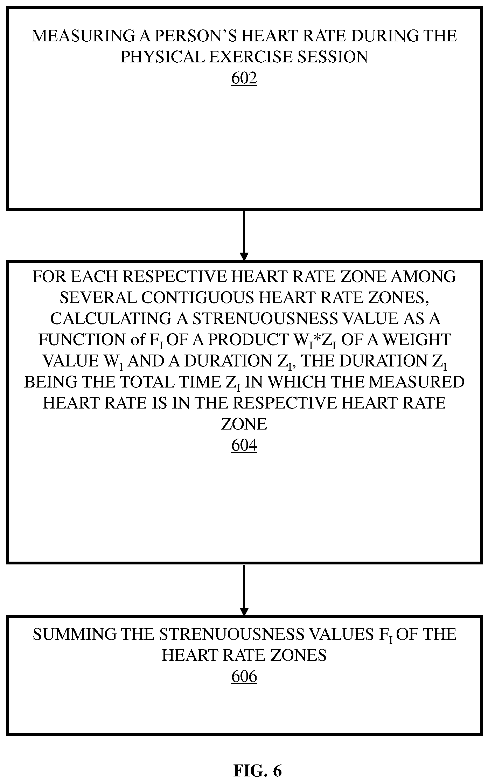

FIG. 6 is a flow diagram that illustrates a method of quantifying a strenuous physical exercise session, in accordance with an implementation of the disclosure. At a step **602,** a person's heart rate during the physical exercise session is measured. At a step **604,** for each respective heart rate zone among several contiguous heart rate zones, a strenuousness as a function fᵢ of a product wᵢ*zᵢ of a weight value wᵢ and a duration zᵢ is caulcualted. The duration zᵢ is being the total time zᵢ in which the measured heart rate is in the respective heart rate zone. At a step **606,** the strenuousness values fi of the heart rate zones are summed to obtain the overall stress.

Thus, the training stress is estimated by first classifying the heart rate sample during training session into different heart rate zones based on user's maximum heart rate. The overall training stress estimation is improved by calculating the fatigue level based on measured performance level during the endurance exercise and by using the calculated fatigue level scaling coefficient to adjust the training stress and recovery time accordingly. Furthermore, the fatigue level scaling coefficient can be adapted individually for each user and thus provide more personalized training stress and recovery time values.

Optionally, the weight value wᵢ is determined based on the heart rate zone and a duration tₜₒₜ of the physical exercise session. Optionally, each strenuousness value is calculated in dependence of a fitness level calculation function flᵢ for the respective heart rate zone. Optionally, the overall training stress is calculated based on the sum of all strenuousness values divided by the total duration tₜₒₜ which is the sum of the time spend in all heart-rate zones. Optionally, the overall training stress is adjusted by a fatigue level coefficient which is an estimate of a fatigue level of the person based on a work metric, such as work to effort ratio, recorded during the session. Optionally, the fatigue level coefficient is also calculated based on the residual and/or added fatigue estimated from the real-time performance level during the training session.

Optionally, the overall training stress is adjusted by a sport type calculation function depending on the type of sports practice in the training session, to account for the physiological strain perceived by the person to quantify the relative intensity and duration of the training session. The overall training stress is calculated according to the equation: $f = \frac{{\sum }_{i} {w}_{i} {z}_{i} {fl}_{i}}{{t}_{tot}} \times flc \times stc ,$ where wᵢ is the weight calculation function for heart rate zone i, zᵢ is the duration of the time spent in heart rate zone i, flᵢ is the fitness level calculation function for heart rate zone i, tₜₒₜ is the sum of the time spent in all heart rate zones, flc is the fatigue level calculation function and stc is the sport type calculation function, and where one or more of flᵢ, flc and stc is set to 1.0. Here, a recovery time for the person is calculated based on the overall training stress.

In an implementation, there is provided a computer program for use in a mobile or wearable fitness tracker device. The computer program product comprises computer-readable code which, when run in a processor will cause the processor to perform the method.

Optionally, the computer program including a non-transitory storage device holding the computer-readable code means.

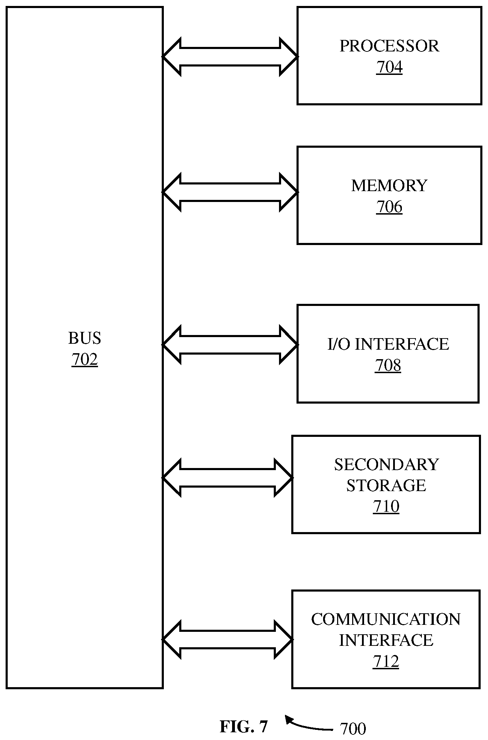

FIG. 7 is an illustration of an exemplary computing arrangement **700** (e.g. a device) in which the various architectures and functionalities of the various previous implementations may be implemented. As shown, the computing arrangement **700** includes at least one processor **704** that is connected to a bus **702,** wherein the computing arrangement **700** may be implemented using any suitable protocol, such as PCI (Peripheral Component Interconnect), PCI-Express, AGP (Accelerated Graphics Port), HyperTransport, or any other bus or point-to-point communication protocol (s). The computing arrangement **700** also includes a memory **706.**

Control logic (software) and data are stored in the memory **706** which may take the form of random-access memory (RAM). In the present description, a single semiconductor platform may refer to a sole unitary semiconductor-based integrated circuit or chip. It should be noted that the term single semiconductor platform may also refer to multi-chip modules with increased connectivity which simulate on-chip modules with increased connectivity which simulate on-chip operation, and make substantial improvements over utilizing a conventional central processing unit (CPU) and bus implementation. Of course, the various modules may also be situated separately or in various combinations of semiconductor platforms per the desires of the user.

The computing arrangement **700** may also include a secondary storage **710.** The secondary storage **710** includes, for example, a hard disk drive and a removable storage drive, representing a floppy disk drive, a magnetic tape drive, a compact disk drive, digital versatile disk (DVD) drive, recording device, universal serial bus (USB) flash memory. The removable storage drive at least one of reads from and writes to a removable storage unit in a well-known manner.

Computer programs, or computer control logic algorithms, may be stored in at least one of the memory **706** and the secondary storage **710.** Such computer programs, when executed, enable the computing arrangement **700** to perform various functions as described in the foregoing. The memory **706,** the secondary storage **710,** and any other storage are possible examples of computer-readable media.

In an implementation, the architectures and functionalities depicted in the various previous figures may be implemented in the context of the processor **704,** a graphics processor coupled to a communication interface **712,** an integrated circuit (not shown) that is capable of at least a portion of the capabilities of both the processor **704** and a graphics processor, a chipset (i.e., a group of integrated circuits designed to work and sold as a unit for performing related functions, etc.).

Furthermore, the architectures and functionalities depicted in the various previous figures may be implemented in the context of a general computer system, a circuit board system, a game console system dedicated for entertainment purposes, an application-specific system. For example, the computing arrangement **700** may take the form of a desktop computer, a laptop computer, a server, a workstation, a game console, an embedded system.

Furthermore, the computing arrangement **700** may take the form of various other devices including, but not limited to a personal digital assistant (PDA) device, a mobile phone device, a smart phone, a television, etc. Additionally, although not shown, the computing arrangement **700** may be coupled to a network (e.g., a telecommunications network, a local area network (LAN), a wireless network, a wide area network (WAN) such as the Internet, a peer-to-peer network, a cable network, or the like) for communication purposes through an I/O interface **708.**

The disclosure improves overall training stress and recovery time estimation by taking into account the measured fatigue level. Sport type coefficient parameter further improves perceived subjective feeling of the exercise stress between different sport types. Personally fitted fatigue level scaling parameters can take into account person's individual physiological characteristics better than general fitness based models in prior art. Also, base training stress calculation is computationally inexpensive.

It should be understood that the arrangement of components illustrated in the figures described are exemplary and that other arrangement may be possible. It should also be understood that the various system components (and means) defined by the claims, described below, and illustrated in the various block diagrams represent components in some systems configured according to the subject matter disclosed herein. For example, one or more of these system components (and means) may be realized, in whole or in part, by at least some of the components illustrated in the arrangements illustrated in the described figures.

In addition, while at least one of these components are implemented at least partially as an electronic hardware component, and therefore constitutes a machine, the other components may be implemented in software that when included in an execution environment constitutes a machine, hardware, or a combination of software and hardware.

Although the disclosure and its advantages have been described in detail, it should be understood that various changes, substitutions, and alterations can be made herein without departing from the scope of the disclosure as defined by the appended claims.

## Claims

1. A method of quantifying strenuousness of a physical exercise session of a person, the method including:
measuring (602) the person's heart rate during the physical exercise session;
for each respective heart rate zone among several contiguous heart rate zones, calculating (604) a strenuousness value fᵢ as a function of a product wᵢ*zᵢ of a weight value wᵢ and a duration zᵢ, the duration zᵢ being a total time zᵢ in which the measured heart rate is in the respective heart rate zone; and
computing (606) a sum of the strenuousness values fᵢ of the heart rate zones,
**characterised in that**
an overall training stress is calculated based on the sum of all strenuousness values divided by the total duration tₜₒₜ which is the sum of the time spent in all heart-rate zones; and
**in that** the overall training stress is adjusted by:
- a fatigue level coefficient which is an estimate of a fatigue level of the person based on a work metric of a performance level recorded during the session; and/or
- a sport type calculation function depending on the type of sports practice in the training session, to quantify the relative intensity and duration of the training session.

2. The method according to claim 1, wherein the weight value wᵢ is determined based on the heart rate zone and a duration tₜₒₜ of the physical exercise session.

3. The method according to claim 1, wherein each strenuousness value fᵢ is calculated in dependence of a fitness level calculation function flᵢ for the respective heart rate zone.

4. The method according to any one of the preceding claims, wherein the fatigue level coefficient is also calculated based on the residual and/or added fatigue estimated from the real-time performance level during the training session.

5. The method according to any one of the preceding claims, wherein the overall training stress is calculated according to the equation $f = \frac{{\sum }_{i} {w}_{i} {z}_{i} {fl}_{i}}{{t}_{tot}} \times flc \times stc$,
where wᵢ is the weight calculation function for heart rate zone i, zᵢ is the duration of the time spent in heart rate zone i , flᵢ is the fitness level calculation function for heart rate zone i, tₜₒₜ is the sum of the time spent in all heart rate zones, flc is the fatigue level calculation function and stc is the sport type calculation function, and where one or more of flᵢ, flc and stc may be set to 1.0.

6. The method according to any one of the preceding claims, comprising the step of calculating a recovery time for the person based on the overall training stress.

7. A computer program for use in a mobile or wearable fitness tracker device, the computer program comprising computer-readable code means which, when run in a processor will cause the processor to perform the method of any one of the preceding claims.

8. The computer program according to claim 7, stored on a non-transitory storage device.

9. A device (300) for calculating an overall training stress resulting from a training session for a person, the device (300) comprising a processor (302) and a program memory (304), the program memory (304) holding a computer program according to claim 7 so that it can be executed in the processor (302).

10. The device (300) according to claim 9, further comprising at least one input for receiving real-time information about the heart rate of the person and optionally at least one of the following types of sensor data for use by the computer program:
- speed sensor data;
- power sensor data;
- accelerometer data; and
- electromyography data.

11. The device (300) according to claim 9 or 10, further comprising at least one input (304) for receiving at least one of the following types of data for use by the computer program:
type of sport performed in the session;
maximum heart rate of the person;
resting heart rate; and
heart rate zones.

12. The device (300) according to claim 9 or 10, further comprising a data memory (308) holding at least one of the following types of data for use by the computer program:
type of sport performed in the session;
maximum heart rate of the person;
resting heart rate; and
heart rate zones.

## Patentansprüche

1. Verfahren zum Quantifizieren der Anstrengung einer körperlichen Übungssitzung einer Person, wobei das Verfahren Folgendes beinhaltet:
Messen (602) der Herzfrequenz der Person während der körperlichen Übungssitzung;
für jede jeweilige Herzfrequenzzone unter mehreren angrenzenden Herzfrequenzzonen, Berechnen (604) eines Anstrengungswertes fᵢ als eine Funktion eines Produkts wᵢ*zᵢ aus einem Gewichtungswert wᵢ und einer Dauer zᵢ, wobei die Dauer zᵢ die Gesamtzeit zᵢ, in der sich die gemessene Herzfrequenz in der jeweiligen Herzfrequenzzone befindet, ist; und
Berechnen (606) einer Summe der Anstrengungswerte fᵢ der Herzfrequenzzonen,
**dadurch gekennzeichnet, dass**
eine Gesamtbelastung beim Training basierend auf der Summe sämtlicher Anstrengungswerte, die durch die Gesamtdauer tₜₒₜ geteilt wird, welche die Summe der in sämtlichen Herzfrequenzzonen verbrachten Zeit ist, berechnet wird; und
dadurch, dass die Gesamtbelastung beim Training durch Folgendes angepasst wird:
- ein Ermüdungsgradkoeffizient, der eine Schätzung eines Ermüdungsgrades der Person basierend auf einer Arbeitsmetrik eines während der Sitzung aufgezeichneten Leistungsniveaus darstellt; und/oder
- eine Sportart-Berechnungsfunktion, die von der Art der sportlichen Betätigung in der Trainingssitzung abhängt, um die relative Intensität und Dauer der Trainingssitzung zu quantifizieren.

2. Verfahren nach Anspruch 1, wobei der Gewichtungswert wᵢ basierend auf der Herzfrequenzzone und einer Dauer tₜₒₜ der körperlichen Übungssitzung bestimmt wird.

3. Verfahren nach Anspruch 1, wobei jeder Anstrengungswert fᵢ in Abhängigkeit von einer Fitnessniveau-Berechnungsfunktion flᵢ für die jeweilige Herzfrequenzzone berechnet wird.

4. Verfahren nach einem der vorhergehenden Ansprüche, wobei der Ermüdungsgradkoeffizient auch basierend auf der Rest- und/oder Zusatzermüdung, die aus dem Echtzeit-Leistungsniveau während der Trainingssitzung geschätzt wird, berechnet wird.

5. Verfahren nach einem der vorhergehenden Ansprüche, wobei die Gesamtbelastung beim Training nach der folgenden Gleichung berechnet wird: $f = \frac{{\sum }_{i} {w}_{i} {z}_{i} {fl}_{i}}{{t}_{tot}} \times flc \times stc$,
wobei wᵢ die Gewichtungsberechnungsfunktion für die Herzfrequenzzone i ist, zᵢ die Dauer der in der Herzfrequenzzone i verbrachten Zeit ist, flᵢ die Fitnessniveau-Berechnungsfunktion für die Herzfrequenzzone i ist, tₜₒₜ die Summe der in sämtlichen Herzfrequenzzonen verbrachten Zeit ist, flc die Ermüdungsgrad-Berechnungsfunktion ist und stc die Sportart-Berechnungsfunktion ist, wobei eine oder mehrere der Funktionen von flᵢ, flc und stc auf 1,0 gesetzt werden können.

6. Verfahren nach einem der vorhergehenden Ansprüche, umfassend den Schritt des Berechnens einer Erholungszeit für die Person basierend auf der Gesamtbelastung beim Training.

7. Computerprogramm zur Verwendung in einer mobilen oder tragbaren Fitness-Tracker-Vorrichtung, wobei das Computerprogramm einen computerlesbaren Code umfasst, der, wenn er in einem Prozessor ausgeführt wird, den Prozessor dazu veranlasst, das Verfahren eines der vorhergehenden Ansprüche durchzuführen.

8. Computerprogramm nach Anspruch 7, das auf einer nichtflüchtigen Speichervorrichtung gespeichert ist.

9. Vorrichtung (300) zum Berechnen einer Gesamtbelastung beim Training, die sich durch eine Übungssitzung für eine Person ergibt, wobei die Vorrichtung (300) einen Prozessor (302) und einen Programmspeicher (304) umfasst, wobei der Programmspeicher (304) ein Computerprogramm nach Anspruch 7 enthält, sodass dieses in dem Prozessor (302) ausgeführt werden kann.

10. Vorrichtung (300) nach Anspruch 9, ferner umfassend mindestens einen Eingang zum Empfangen von Echtzeitinformationen über die Herzfrequenz der Person und optional mindestens eine der folgenden Arten von Sensordaten zur Verwendung durch das Computerprogramm:
- Daten des Geschwindigkeitssensors;
- Daten des Leistungssensors;
- Daten des Beschleunigungsmessers; und
- Elektromyographiedaten.

11. Vorrichtung (300) nach Anspruch 9 oder 10, ferner umfassend mindestens einen Eingang (304) zum Empfangen mindestens einer der folgenden Arten von Daten zur Verwendung durch das Computerprogramm:
Art der in der Sitzung ausgeübten Sportart;
maximale Herzfrequenz der Person;
Ruhepuls; und
Herzfrequenzzonen.

12. Vorrichtung (300) nach Anspruch 9 oder 10, ferner umfassend einen Datenspeicher (308), der mindestens eine der folgenden Arten von Daten zur Verwendung durch das Computerprogramm enthält:
Art der in der Sitzung ausgeübten Sportart;
maximale Herzfrequenz der Person;
Ruhepuls; und
Herzfrequenzzonen.

## Revendications

1. Procédé de quantification de l'intensité d'une séance d'exercice physique pour un individu, le procédé comportant :
la mesure (602) de la fréquence cardiaque de la personne pendant la séance d'exercice physique ;
pour chaque zone de fréquence cardiaque respective parmi plusieurs zones de fréquence cardiaque contiguës, le calcul (604) d'une valeur d'intensité fᵢ en tant que fonction d'un produit wᵢ*zᵢ d'une valeur de poids wᵢ et d'une durée zᵢ, la durée zᵢ étant un temps total zᵢ pendant lequel la fréquence cardiaque mesurée se trouve dans la zone de fréquence cardiaque respective ; et
le calcul (606) d'une somme des valeurs d'intensité fᵢ des zones de fréquence cardiaque,
**caractérisé en ce que**
l'intensité globale de l'entraînement est calculée en divisant la somme de toutes les valeurs d'intensité par la durée totale tₜₒₜ, qui correspond à la somme du temps passé dans toutes les zones de fréquence cardiaque ; et
**en ce que** l'intensité globale de l'entraînement est ajustée par :
- un coefficient de niveau de fatigue qui est une estimation du niveau de fatigue de l'individu basée sur une mesure de travail d'un niveau de performance enregistrée au cours de la session ; et/ou
- une fonction de calcul du type de sport, dépendant du type de pratique sportive lors de la séance d'entraînement, afin de quantifier l'intensité relative et la durée de la séance d'entraînement.

2. Procédé selon la revendication 1, dans lequel la valeur de poids wᵢ est déterminée en fonction de la zone de fréquence cardiaque et d'une durée tₜₒₜ de la séance d'exercice physique.

3. Procédé selon la revendication 1, dans lequel chaque valeur d'intensité fᵢ est calculée en fonction d'une fonction de calcul du niveau de forme physique flᵢ pour la zone de fréquence cardiaque respective.

4. Procédé selon l'une quelconque des revendications précédentes, dans lequel le coefficient de niveau de fatigue est également calculé sur la base de la fatigue résiduelle et/ou ajoutée estimée à partir du niveau de performance en temps réel pendant la séance d'entraînement.

5. Procédé selon l'une quelconque des revendications précédentes, dans lequel l'intensité globale de l'entraînement est calculée selon l'équation $f = \frac{{\sum }_{i} {w}_{i} {z}_{i} {fl}_{i}}{{t}_{tot}} \times flc \times stc$,
où wᵢ est la fonction de calcul de poids pour la zone de fréquence cardiaque i, zᵢ est la durée du temps passé dans la zone de fréquence cardiaque i, flᵢ est la fonction de calcul du niveau de forme physique pour la zone de fréquence cardiaque i, tₜₒₜ est la somme du temps passé dans toutes les zones de fréquence cardiaque, flc est la fonction de calcul du niveau de fatigue et stc est la fonction de calcul du type de sport, et où l'un ou plusieurs des éléments flᵢ, flc et stc peuvent être fixés à 1,0.

6. Procédé selon l'une quelconque des revendications précédentes, comprenant l'étape de calcul d'un temps de récupération pour l'individu sur la base de l'intensité globale de l'entraînement.

7. Programme informatique destiné à être utilisé dans un dispositif de suivi de la forme physique mobile ou portable, le programme informatique comprenant des moyens de code lisibles par ordinateur qui, lorsqu'ils sont exécutés dans un processeur, amèneront le processeur à réaliser le procédé selon l'une quelconque des revendications précédentes.

8. Programme informatique selon la revendication 7, stocké sur un dispositif de stockage non transitoire.

9. Dispositif (300) pour calculer une intensité globale d'entraînement résultant d'une séance d'entraînement pour un individu, le dispositif (300) comprenant un processeur (302) et une mémoire de programme (304), la mémoire de programme (304) contenant un programme informatique selon la revendication 7 afin qu'il puisse être exécuté dans le processeur (302).

10. Dispositif (300) selon la revendication 9, comprenant en outre au moins une entrée pour recevoir des informations en temps réel sur la fréquence cardiaque de l'individu et, éventuellement, au moins l'un des types de données de capteur suivantes destinées à être utilisées par le programme informatique :
- données du capteur de vitesse ;
- données du capteur d'énergie ;
- données de l'accéléromètre ; et
- données électromyographiques.

11. Dispositif (300) selon la revendication 9 ou 10, comprenant en outre au moins une entrée (304) destinée à recevoir au moins l'un des types de données suivantes destinées à être utilisées par le programme informatique :
type de sport pratiqué lors de la séance ;
fréquence cardiaque maximale de l'individu ;
fréquence cardiaque au repos ; et
zones de fréquence cardiaque.

12. Dispositif (300) selon la revendication 9 ou 10, comprenant en outre une mémoire de données (308) contenant au moins l'un des types de données suivantes destinées à être utilisées par le programme informatique :
type de sport pratiqué lors de la séance ;
fréquence cardiaque maximale de l'individu ;
fréquence cardiaque au repos ; et
zones de fréquence cardiaque.
